# EUROPEAN PATENT SPECIFICATION

(11) **EP 3 875 073 B1**
(45) Date of publication and mention of the grant of the patent: **31.12.2025**
(21) Application number: 19879410.9
(22) Date of filing: 30.10.2019
(51) Int. Cl.: A61K 8/06, A61K 8/34, A61K 8/37, A61K 8/67, A61K 8/73, A61K 8/92, A61Q 19/00

(54) **OIL-IN-WATER-TYPE SKIN EXTERNAL AGENT**
EXTERNES ÖL-IN-WASSER-HAUTPFLEGEMITTEL
AGENT EXTERNE POUR LA PEAU DE TYPE HUILE-DANS-EAU

(30) Priority: 02.11.2018 JP 2018207721
(43) Date of publication of application: 08.09.2021
(73) Proprietor: Resonac Corporation, Tokyo (JP)
(72) Inventor: MISHINA Natsuno, Tokyo 105-8518 (JP); ITO Naoko, Tokyo 105-8518 (JP); HATANO Manami, Tokyo 105-8518 (JP)
(74) Representative: Strehl & Partner mbB
(86) International application number: PCT/JP2019/042565
(87) International publication number: WO 2020/090881

(56) References cited:
- WO-A1-2015/033757
- JP-A- 2003 081 736
- JP-A- 2010 512 348
- JP-A- 2014 141 432
- JP-A- 2015 027 953
- JP-A- 2015 151 359
- JP-A- 2018 123 129

## Description

### [Technical Field]

The present invention relates to an oil-in-water-type skin external agent.

### [Background Art]

Tocopherol phosphate ester and a salt thereof are vitamin E derivatives which have acquired water solubility by introducing a phosphate group into vitamin E, and exhibit an excellent effect of preventing rough skin. Tocopherol phosphate ester and a salt thereof have been shown to remove active oxygen, to moisturize, to retain water, to have anti-inflammatory effect, and the like, and are incorporated into various cosmetic formulations.

In the related art, as cosmetics, oil-in-water-type emulsified compositions such as emulsion and cream have been widely used. In order to keep the emulsified dispersion of oil stable in the oil-in-water-type emulsified composition, it is necessary to add a surfactant. In addition, it is also known that a thickener is effective for stabilizing the emulsification of oil. For example, Patent Documents 1 and 2 disclose oil-in-water-type emulsified compositions which include a water-soluble polymer in which agar and xanthan gum are mixed at a specific mass ratio and which do not require the use of a surfactant. Patent Document 3 discloses an oil-in-water-type emulsion including a thickener or a gelling agent.

In addition, it is required for cosmetics not only to have excellent preparation stability but also to exhibit an aesthetic appearance. Cosmetics which exhibit a special appearance include preparations containing oil droplets which can be visually confirmed. For example, Patent Document 4 discloses a spray-type ultraviolet blocking agent containing visible oil droplets having a particle size of approximately 0.1 to 10 mm. Patent Document 5 discloses a body spray containing pearl-like oil droplets having a particle size of approximately 1 to 10 mm. Patent Document 6 discloses a two-phase liquid cosmetic containing oil droplets having a particle size of approximately 1 to 5 mm. Emulsification systems thereof are stabilized by solid particles adsorbed on the interface between two types of incompatible liquids.

### [Citation List]

### [Patent Document]

[Patent Document 1]
   Japanese Unexamined Patent Application, First Publication No. 2015-145416
[Patent Document 2]
   PCT International Publication No. WO2011/111854
[Patent Document 3]
   Published Japanese Translation No. 2009-516584 of the PCT International Publication
[Patent Document 4]
   United States Patent Application, Publication No. 2017/0189279
[Patent Document 5]
   United States Patent No. 6270782
[Patent Document 6]
   United States Patent No. 4767741
JP 2018 123129 A discloses, in an example, a skin care cream composition comprising a 0.1 wt-% tocopherol phosphate ester, 0.1 wt-% gellan gum, 7.0 wt-% glycerin, 2 wt.-% jojoba oil, 3.0 wt-% tri(caprylic acid / capric acid ) glyceryl (Neosolue-MCT: Nippon Fine Chemical Co., Ltd.) and water.

### [Summary of Invention]

### [Technical Problem]

However, an oil-in-water-type cosmetic in which tocopherol phosphate ester and/or a salt thereof are blended as an active ingredient and visible oil droplets are dispersed has not been known so far. In addition, in the cosmetics disclosed in Patent Documents 4 to 6, oil droplets are stabilized by solid particles, and no oil-in-water-type skin external agent in which visible oil droplets are formed regardless of solid particles has been reported.

Therefore, an object of the present invention is to provide an oil-in-water-type skin external agent containing tocopherol phosphate ester and/or a salt thereof as an active ingredient, in which oil droplets that are visible to the naked eye are dispersed and excellent aesthetic appearance is exhibited.

### [Solution to Problem]

The present invention is set out in the appended claims.

### [Advantageous Effects of Invention]

According to the present invention, an oil-in-water-type skin external agent containing tocopherol phosphate ester and/or a salt thereof as an active ingredient, in which oil droplets that are visible are dispersed and excellent aesthetic appearance is exhibited, is provided.

### [Description of Embodiments]

The present invention provides an oil-in-water-type skin external agent. The oil-in-water-type skin external agent of the present embodiment contains the following components (a) to (f).
(a) 0.1 to 3.0 % by mass of sodium tocopherol phosphate;
(b) 0.01% to 1.0% by mass of gellan gum;
(c1) 0.2 to 6.0% by mass of 1,2-hexanediol;
(c2) 0.9% to 4.0% by mass of 2-methyl-1,3-propanediol and 0.13% to 1.6% by mass of glycerin;
(d) 0.1% to 2.0% by mass of propanediol dicaprylate/dicaprate and 0.1% to 2.0% by mass of caprylic/capric triglyceride;
(e) water; and
(f) 0.01% to 1.0% by mass of tamarind gum.

In the present specification, the "oil-in-water-type skin external agent" refers to a skin external agent in a state in which oil droplets are dispersed in the aqueous phase. Examples of a particle size of the oil droplets include 5 µm or more. In the skin external agent of the present embodiment, from the viewpoint of aesthetic appearance, examples of an average particle size of the oil droplets include approximately 5 to 450 µm. The average particle size of the oil droplets is preferably 50 to 300 µm and more preferably 100 to 160 µm. The average particle size of the oil droplets can be measured with a digital microscope. The average particle size is an arithmetic mean value of particle size measurement values of 200 or more oil droplets. The oil droplets dispersed in the aqueous phase are preferably visible to the naked eye.

In the present specification, the "skin external agent" means a composition used by being applied or affixed to the skin, and includes pharmaceuticals, quasi-drugs, cosmetics, and the like.

### [Component (a)]

The component (a) is a salt of a tocopherol phosphate ester, namely sodium tocopherol phosphate (TPNa). Tocopherol phosphate ester is a compound in which a phosphate group is introduced into a hydroxy group of tocopherol. Tocopherol phosphate ester and a salt thereof have been confirmed to have active oxygen removing ability, moisturizing effect, water retention ability, anti-inflammatory effect, skin color improving effect, and the like. Therefore, by blending tocopherol phosphate ester and/or a salt thereof, the above-described effects can be imparted to the skin external agent.

Examples of tocopherol phosphate ester in the component (a) include a compound represented by Formula (1). 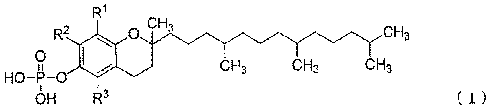 [in the formula, R¹, R², and R³ each independently represent a hydrogen atom or a methyl group]

Tocopherol phosphate ester includes, depending on the types of R¹, R², and R³ in Formula (1), α-tocopherol phosphate ester (R¹, R², R³ = CH₃), β-tocopherol phosphate ester (R¹, R³ = CH₃, R² = H), γ-tocopherol phosphate ester (R¹, R² = CH₃, R³ = H), δ-tocopherol phosphate ester (R¹ = CH₃, R², R³ = H), ζ₂-tocopherol phosphate ester (R², R³ = CH₃, R¹ = H), η-tocopherol phosphate ester (R² = CH₃, R¹, R³ = H), and the like.

Tocopherol phosphate ester in the component (a) is not particularly limited, and may be any one of these tocopherol phosphate esters. Among these, α-tocopherol phosphate ester or γ-tocopherol phosphate ester is preferable, and α-tocopherol phosphate ester is more preferable.

Since the compound represented by Formula (1) has an asymmetric carbon atom at the 2-position of the chromane ring, the compound represented by Formula (1) includes a stereoisomer of d-form and l-form, and dl-form. Tocopherol phosphate ester in the component (a) may be any one of these stereoisomers, but the dl-form is preferable.

Among these, as tocopherol phosphate ester, dl-α-tocopherol phosphate ester or dl-γ-tocopherol phosphate ester is preferable, and dl-α-tocopherol phosphate ester is more preferable.

The salt of tocopherol phosphate ester in the component (a) is a sodium salt. Since the sodium salt has a high solubility in water and is in powder form, the sodium salt of tocopherol phosphate ester has advantages that it is easy to handle.

Examples of a preferred aspect of tocopherol phosphate ester in the component (a) include a sodium salt of α-tocopherol phosphate ester, a sodium salt of γ-tocopherol phosphate ester, a sodium salt of dl-α-tocopherol phosphate ester, and a sodium salt) of dl-γ-tocopherol phosphate ester.

The sodium salt of dl-α-tocopherol phosphate ester is commercially available from SHOWA DENKO K.K. as a product name of TPNa (registered trademark) (labeling name: Na tocopheryl phosphate). The TPNa is an exemplary example as a preferred example of the component (a).

The content of the component (a) in the oil-in-water-type skin external agent of the present embodiment is 0.1% to 3.0% by mass. In a case where the content of the component (a) is 0.1% by mass or more, the ability of tocopherol phosphate ester or a salt thereof (active oxygen removing ability, moisturizing effect, water retention ability, anti-inflammatory effect, skin color improving effect, and the like) is sufficiently exhibited. In addition, in a case where the content of the component (a) is 3.0% by mass or less, it is easy to balance with other components. In addition, by setting the content of the component (a) within the above-described range, oil droplets having an appropriate particle size for an excellent aesthetic appearance can be formed.

The content of the component (a) in the oil-in-water-type skin external agent of the present embodiment is more preferably 0.3% to 2.5% by mass and still more preferably 0.5% to 2.0% by mass.

Tocopherol phosphate ester and the salt thereof can be produced by a known production method, for example, methods described in Japanese Unexamined Patent Application, First Publication No. S59-44375, PCT International Publication No. WO1997/14705, and the like.

For example, tocopherol phosphate ester can be obtained by reacting tocopherol dissolved in a solvent with a phosphorylating agent such as phosphorus oxychloride and appropriately purifying the reactant after completion of the reaction. Furthermore, the salt of tocopherol phosphate ester can be obtained by neutralizing the obtained tocopherol phosphate ester with a metal oxide such as magnesium oxide or a metal hydroxide such as sodium hydroxide, or ammonium hydroxide or alkylammonium hydroxide.

Hereinafter, tocopherol phosphate ester and the salt thereof may be collectively referred to as "tocopherol phosphate ester and the like".

The sodium tocopherol phosphate in the oil-in-water-type skin external agent of the present embodiment functions as an active ingredient as described above, and also acts as a surfactant for stabilizing the oil component in the aqueous phase. As a result, the oil component in the aqueous phase can be kept stable even in a case where the addition amount of surfactant other than tocopherol phosphate ester or the salt thereof is reduced. In addition, in the oil-in-water-type skin external agent of the present embodiment, it does not need to contain a surfactant other than the sodium tocopherol phosphate ester. However, in the oil-in-water-type skin external agent of the present embodiment, other surfactants may be contained in addition to the sodium tocopherol phosphate ester.

### [Component (b)]

The component (b) is gellan gum. In the oil-in-water-type skin external agent of the present embodiment, the component (b) is used as a thickener. By blending the component (b), it is possible to impart an appropriate viscosity (thickening) to the oil-in-water-type skin external agent and to form the oil droplets having an appropriate particle size for an excellent aesthetic appearance.

Gellan gum is a polysaccharide produced by Sphingomonas elodea, and is composed of tetrasaccharides of glucose, glucuronic acid, glucose, and L-rhamnose, which are repeatedly and linearly linked. Gellan gum includes native gellan gum containing a high acyl group and deacylated gellan gum in which gellan gum is deacylated. Any gellan gum may be used as the component (b), but it is preferable to use deacylated gellan gum.

As gellan gum, commercially available gellan gum can be used without particular limitation. Examples of the commercially available gellan gum include KELCOGEL (registered trademark) Gellan Gum CG-LA (manufactured by CP Kelco).

The content of the component (b) in the oil-in-water-type skin external agent of the present embodiment is 0.01% to 1.0% by mass. In a case where the content of the component (b) is within the above-described preferred range, an appropriate thickening is imparted to the oil-in-water-type skin external agent. In addition, creaming (phenomenon in which the oil phase floats in the upper layer) is sufficiently suppressed, and the oil droplets having an appropriate particle size for an excellent aesthetic appearance can be stably maintained.

The content of the component (b) in the oil-in-water-type skin external agent of the present embodiment is more preferably 0.02% to 0.5% by mass, and particularly preferably 0.03% to 0.1% by mass.

### [Component (c)]

The component (c) is is a combination of a "component (c1)") which is used for dissolving the above-described component (a), and of a "component (c2)") which is used for exhibiting thickening action of the thickener.

The component (c1) is 1,2-hexanediol from the viewpoint of transparency of the oil-in-water-type skin external agent.

The component (c2) includes 2-methyl-1,3-propanediol and glycerin.

2-methyl-1,3-propanediol and glycerin are used in combination as the component (c2) from the viewpoint of improving the transparency of the oil-in-water-type skin external agent and suppressing creaming, and the proportion (mass ratio) of 2-methyl-1,3-propanediol and glycerin is preferably 2-methyl-1,3-propanediol:glycerin = 1:0.03 to 1:1, and more preferably 2-methyl-1,3-propanediol:glycerin = 1:0.1 to 1:0.6.

The content of the component (c) in the oil-in-water-type skin external agent of the present embodiment is preferably 1.5% to 10.0% by mass. The content of the above-described component (c1) in the oil-in-water-type skin external agent of the present embodiment is 0.2% to 6.0% by mass and more preferably 2.0% to 5.0% by mass. In addition, the content of the above-described component (c2) in the oil-in-water-type skin external agent of the present embodiment is preferably 1.5% to 4.5% by mass and more preferably 2.0% to 4.0% by mass. In a case where the contents of the component (c1) and the component (c2) are within the above-described preferred range, it is possible to impart an appropriate viscosity to the oil-in-water-type skin external agent and to stably maintain the oil droplets having an appropriate particle size for an excellent aesthetic appearance.

### [Component (d)]

The component (d) is a combination of specific esters (hereinafter, may be referred to as "fatty acid ester") of fatty acids having 6 to 12 carbon atoms and polyhydric alcohols. In the oil-in-water-type skin external agent of the present embodiment, the component (d) is used as a component of the oil droplets.

The fatty acid ester as the component (d) preferably has a specific gravity close to 1. For example, the specific gravity is preferably approximately 0.80 to 0.99, and more preferably approximately 0.85 to 0.99. By using a component (d) having a specific gravity within the above-described preferred range, it is possible to prepare an oil-in-water-type skin external agent having an aesthetic appearance in which oil droplets are dispersed in the aqueous phase.

The fatty acid ester as the component (d) is a combination of propanediol dicaprylate/dicaprate and caprylic/capric triglyceride. Propanediol dicaprylate/dicaprate is a diester of caprylic acid and capric acid, and propanediol. Further, caprylic/capric triglyceride is a triester of caprylic acid and capric acid, and glycerin.

As the fatty acid ester, a commercially available product can be used without particular limitation. For example, as a commercially available product of propanediol dicaprylate/dicaprate, SALACOS (registered trademark) PR-85 (manufactured by The Nisshin OilliO Group, Ltd.) is an exemplary example; as a commercially available product of caprylic/capric triglyceride, NIKKOL (registered trademark) Triester F-810 (manufactured by Nikko Chemicals Co., Ltd.) is an exemplary example.

The content of the component (d) in the oil-in-water-type skin external agent of the present embodiment is 0.2% to 4.0% by mass. In a case where the content of the component (d) is within the above-described preferred range, oil droplets having an appropriate particle size can be appropriately dispersed in the aqueous phase, and the aesthetic appearance is improved. The content of the component (d) in the oil-in-water-type skin external agent of the present embodiment is more preferably 0.6% to 2.0% by mass. It is still more preferably 0.8% to 1.2% by mass.

From the viewpoint of controlling the particle size of oil droplets, a combination of propanediol dicaprylate/dicaprate and caprylic/capric triglyceride is used. The content of propanediol dicaprylate/dicaprate in the oil-in-water-type skin external agent of the present embodiment is 0.1% to 2.0% by mass and more preferably 0.3% to 1.0% by mass. In addition, the content of caprylic/capric triglyceride in the oil-in-water-type skin external agent of the present embodiment is 0.1% to 2.0% by mass and more preferably 0.3% to 1.0% by mass. In addition, the proportion (mass ratio) of propanediol dicaprylate/dicaprate and caprylic/capric triglyceride is preferably propanediol dicaprylate/dicaprate:caprylic/capric triglyceride = 1:0.5 to 1:2, and more preferably 1:0.7 to 1:1.5.

### [Component (e)]

The component (e) is water. The water may be water having a grade that can be used in cosmetics, such as purified water. The component (e) forms an aqueous phase in the oil-in-water-type skin external agent of the present embodiment. In addition, the component (e) is also used as a solvent for dissolving or mixing the above-described components (a) to (d) and other components described later, which may be added in some cases.

The content of the component (e) is the balance of the total amount of the above-described components (a) to (d) and other components described later.

### (Tamarind gum: component (f))

The oil-in-water-type skin external agent of the present embodiment p contains tamarind gum (hereinafter, may be referred to as a "component (f)"). Thereby, the creaming of the oil-in-water-type skin external agent is suppressed more effectively, and stability is improved.

Tamarind gum is a polysaccharide obtained from tamarind seeds. As tamarind gum, commercially available tamarind gum can be used without particular limitation. Examples of the commercially available tamarind gum include GLYLOID (registered trademark) 6C (DSP GOKYO FOOD & CHEMICAL Co., Ltd.).

The content of tamarind gum in the oil-in-water-type skin external agent is 0.01% to 1.0% by mass. In a case where the content of tamarind gum is within the above-described preferred range, the effect of suppressing creaming is improved. The content of tamarind gum in the oil-in-water-type skin external agent of the present embodiment is more preferably 0.03% to 0.1% by mass.

From the viewpoint of the effect of suppressing creaming, the proportion (mass ratio) of gellan gum as the component (b) and tamarind gum is preferably gellan gum:tamarind gum = 1:0.5 to 1:2, and more preferably gellan gum:tamarind gum = 1:0.7 to 1:1.5.

### [Optional component]

The oil-in-water-type skin external agent of the present embodiment may include other components in addition to the above-described components (a) to (f). Examples of other components include sodium gluconate and sodium chloride.

### (Sodium gluconate)

The oil-in-water-type skin external agent of the present embodiment may contain sodium gluconate. By containing sodium gluconate, it can be expected to have a chelating action of capturing metal ions and an action as a skin conditioning agent.

In a case where the oil-in-water-type skin external agent of the present embodiment contains sodium gluconate, the content of sodium gluconate in the oil-in-water-type skin external agent is preferably 0.01% to 1.0% by mass and more preferably 0.1% to 1.0% by mass.

### (Sodium chloride)

The oil-in-water-type skin external agent of the present embodiment may contain sodium chloride.

In a case where the oil-in-water-type skin external agent of the present embodiment contains sodium chloride, the content of sodium chloride in the oil-in-water-type skin external agent is preferably 0.01% to 1.0% by mass and more preferably 0.1% to 1.0% by mass.

### (Other)

In addition to those described above, the oil-in-water-type skin external agent of the present embodiment may contain any component generally used in cosmetics as long as the effects of the present invention are not impaired.

Examples of such components include preservatives, antibacterial agents, whitening agents, vitamins and derivatives thereof, antiphlogistic agents, anti-inflammatory agents, blood circulation promoters, hormones, anti-wrinkle agents, anti-aging agents, tightening agents, cold sense agents, warm sense agents, wound-healing promoters, abirritants, painkillers, cell activators, plant/animal/microbial extracts, antipruritic agents, keratin exfoliating/dissolving agents, astringent agents, enzymes, nucleic acids, fragrances, coloring agents, colorants, antiphlogistic analgesics, antifungal agents, antihistamines, antibiotics, antibacterial substances, crude drugs, antipruritic drugs, keratin softening and stripping agents, antiseptic and bactericidal agents, antioxidants, pH adjusters, and additives. Specific examples of these components include those described in Japanese Unexamined Patent Application, First Publication No. 2016-50196. One kind of the other components may be used alone, or two or more kinds thereof may be used in combination.

Examples of the preservative include benzoic acid, sodium benzoate, undecylenic acid, salicylic acid, sorbic acid, potassium sorbate, dehydroacetic acid, sodium dehydroacetate, isobutyl para-hydroxybenzoate, isopropyl para-hydroxybenzoate, ethyl para-hydroxybenzoate, butyl para-hydroxybenzoate, propyl para-hydroxybenzoate, benzyl para-hydroxybenzoate, methyl para-hydroxybenzoate, sodium methyl para-hydroxybenzoate, phenoxyethanol, photosensitizer 101, photosensitizer 201, and photosensitizer 401. However, the preservative is not limited thereto. One kind of the preservative may be used alone, or two or more kinds thereof may be used in combination. Phenoxyethanol is an exemplary example as a preferred preservative.

Examples of the pH adjuster include citric acid, sodium hydroxide, potassium hydroxide, and triethanolamine. However, the pH adjuster is not limited thereto. One kind of the pH adjuster may be used alone, or two or more kinds thereof may be used in combination. Citric acid is an exemplary example as a preferred pH adjuster.

Examples of the coloring agent, colorant, dye, or pigment include nitro coloring agents; azo coloring agents; nitroso coloring agents; triphenylmethane coloring agents; xanthene coloring agents; quinoline coloring agents; anthraquinone coloring agents; indigo coloring agents; pyrene coloring agents; phthalocyanine coloring agents; natural coloring agents such as flavonoid, quinone, porphyrin, water-soluble annatto, powdered squid ink, caramel, guaiazulene, gardenia blue, gardenia yellow, cochineal, shikonin, copper chlorophyrin sodium, paprika coloring agent, carthamus red, carthamus yellow, laccaic acid, and riboflavin butyrate; carbon black; yellow iron oxide; black iron oxide; red iron oxide; ferric ferrocyanide; ultramarine; zinc oxide; chromium oxide; titanium oxide; black titanium oxide; zirconium oxide; chromium hydroxide; alumina; magnesium oxide; barium sulfate; aluminum hydroxide; calcium carbonate; lithium cobalt titanate; manganese violet; and pearl pigment. However, the coloring agent, colorant, dye, or pigment is not limited thereto. One kind of the coloring agent, colorant, dye, or pigment may be used alone, or two or more kinds thereof may be used in combination.

Specific examples of the azo coloring agent include Sudan III.

The pH of the oil-in-water-type skin external agent of the present embodiment is preferably approximately 5.5 to 8.5 and more preferably approximately 6.5 to 8.5. In a case where the pH is within the above-described preferred range, irritation to the skin can be reduced and stability of the preparation can be maintained. The pH is a value at 25°C and can be measured with a pH meter.

The pH of the oil-in-water-type skin external agent of the present embodiment can be adjusted by using the pH adjuster or the like described as an example above.

The formulation of the oil-in-water-type skin external agent of the present embodiment is described below.
(a) 0.1% to 3.0% by mass (preferably 0.5% to 2.0% by mass) of sodium tocopherol phosphate (TPNa)
(b) 0.01% to 1.0% by mass (preferably 0.03% to 0.1% by mass) of gellan gum
(c1) 0.2% to 6.0% by mass (preferably 2.0% to 4.0% by mass) of 1,2-hexanediol
(c2) 0.9% to 4.0% by mass (preferably 1.2% to 3.6% by mass) of 2-methyl-1,3-propanediol and 0.13% to 1.6% by mass (preferably 0.18% to 1.4% by mass) of glycerin
(d) 0.1% to 2.0% by mass (preferably 0.3% to 1.0% by mass) of propanediol dicaprylate/dicaprate and 0.1% to 2.0% by mass (preferably 0.3% to 1.0% by mass) of caprylic/capric triglyceride
(e) water
(f) 0.01% to 1.0% by mass (preferably 0.03% to 0.1% by mass) of tamarind gum

### [Production method]

The oil-in-water-type skin external agent of the present embodiment can be produced by mixing the above-described components (a) to (f), and optionally adding and mixing other components. A specific example of the method for producing the oil-in-water-type skin external agent of the present embodiment is described below, but the present invention is not limited thereto.

First, the component (a) is dissolved in a part of the component (c) (preferably, the component (c1)), and water is appropriately added thereto to prepare a solution of the component (a). In addition, the component (b) and a part of the component (c) (preferably, the component (c2)) are mixed with each other, water is appropriately added thereto, and the mixture is stirred while heating at approximately 60°C to 80°C (preferably, approximately 70°C) to prepare a solution of the component (b). Next, the solution of the component (a) and the solution of the component (b) prepared above are mixed with each other and left to stand until gelling. After gelling, the gel is broken by stirring, and the component (d) is added dropwise thereto while stirring slowly, whereby the oil-in-water-type skin external agent of the present embodiment can be obtained.

Tamarind gum (component (f)) may be mixed with a part of the component (c) (preferably, the component (c2)) together with gellan gum as the component (b), thereby preparing a dispersion liquid of gellan gum and tamarind gum.

The method for breaking the gel is not particularly limited, and the gel can be broken by, for example, stirring at approximately 300 rpm using a three-one motor (registered trademark) and breaking until a uniform state is obtained.

The method for stirring in a case of adding dropwise the component (d) may be manual stirring or mechanical stirring. From the viewpoint of stable production, mechanical stirring is preferable. As a stirrer used for the mechanical stirring, for example, a three-one motor, a pencil mixer, or the like can be used. The shape of the stirring blade of the stirrer is not particularly limited, and for example, a paddle type, an anchor type, a propeller type, or the like can be used. The rotation speed of the stirrer is, for example, 50 rpm to 200 rpm, preferably 80 rpm to 180 rpm, and more preferably 100 rpm to 150 rpm.

The method for stirring in steps other than the step of adding dropwise the component (d) may be manual stirring or mechanical stirring. From the viewpoint of stable production, mechanical stirring is preferable. As the stirrer used for the mechanical stirring, for example, a device including a motor, a stir bar, and a blade, such as a three-one motor, can be used. The shape of the blade of the stirrer is not particularly limited, and for example, a paddle type, an anchor type, a propeller type, or the like can be used.

In a case of blending an optional component other than the components (a) to (f), it is sufficient that the optional component is appropriately added at any time of the above-described steps, depending on the type of the optional component.

In a case of blending sodium gluconate, sodium gluconate may be dissolved in water, and the water may be added to the solution of the component (b). Alternatively, sodium gluconate may be added to the solution of (b). In addition, in a case of blending sodium chloride, sodium chloride may be dissolved in water, and the water may be added to a mixed solution of the solution of the component (a) and the solution of the component (b). Other additives such as preservative and coloring agent may be added to the mixed solution of the solution of the component (a) and the solution of the component (b) after gelling.

The oil-in-water-type skin external agent of the present embodiment can be suitably used as cosmetics such as lotion, serum, and beauty essence. In addition, as a dosage form, the oil-in-water-type skin external agent of the present embodiment can be suitably used as a spray agent, a mist agent, and the like.

For example, in a case where the oil-in-water-type skin external agent of the present embodiment is used as a spray agent, by a simple operation, oil and water can be applied to the skin at the same time and in a uniform composition. In addition, since the oil-in-water-type skin external agent of the present embodiment contains the thickener, the oil-in-water-type skin external agent of the present embodiment does not drip in a case of being applied to the skin, and the adhesion of the blended component to the skin is improved.

Since the oil-in-water-type skin external agent of the present embodiment contains the component (a), the oil-in-water-type skin external agent of the present embodiment has effects such as active oxygen removing ability, moisturizing effect, water retention ability, anti-inflammatory effect, and skin color improving effect. In addition, since the oil-in-water-type skin external agent of the present embodiment contains the components (b) to (e), the oil-in-water-type skin external agent of the present embodiment has an aesthetic appearance in which oil droplets having appropriate size are dispersed in the aqueous phase. Furthermore, since the oil-in-water-type skin external agent of the present embodiment contains the component (b) and the component (c), the oil-in-water-type skin external agent of the present embodiment has an appropriate thickening and has a high moisturizing effect. In addition, by containing the component (f) in addition to the above-described components (a) to (e), the creaming is suppressed and the stability is improved.

Due to the effects of the components described above, according to the present invention, an oil-in-water-type skin external agent which has the effect of improving skin function from tocopherol phosphate ester and the like, and has an excellent aesthetic appearance, is provided.

### [Examples]

Hereinafter, the present invention will be described with reference to Examples, but the present invention is not limited to the following Examples.

### (Examples 1 to 8 (reference examples) and Comparative Examples 1 to 3)

### [Production of oil-in-water-type skin external agent]

According to compositions shown in Tables 1 to 3, oil-in-water-type skin external agents of Examples 1 to 8 and Comparative Examples 1 to 3 were produced. The units of the numerical values shown in Tables 1 to 3 are % by mass based on the mass of each skin external agent.

Materials A to E shown in Tables 1 to 3 were prepared as follows to produce oil-in-water-type skin external agents of each example.

First, regarding the material B, gellan gum was dispersed in 2-methyl-1,3-propanediol to prepare a dispersion liquid B. Purified water was added thereto and the solution was heated to 80°C, and then the material A was added thereto to prepare a solution AB including the material A and the material B. Next, the materials C were mixed with each other, purified water was added thereto, and the mixture was stirred to prepare a solution C. The material D was dissolved in purified water to prepare a solution D.

The solution AB was maintained at a temperature of 60°C or higher, the solution C was added thereto, and then the solution D was added thereto while stirring. The mixture was left to stand until it became approximately room temperature, and after the surface thereof gelled, the mixture was stirred vigorously for several minutes to break the gel to obtain a liquid gel. Thereafter, as necessary, a preservative (phenoxyethanol) was added thereto, and the pH thereof was adjusted to 5.5 to 6.5 with 10% citric acid.

The material E was added dropwise to the liquid gel while stirring the liquid gel at approximately 150 rpm using a three-one motor (HEIDON, FBL1200, Shinto Scientific Co., Ltd.).

### [Evaluation of oil droplets]

With regard to the oil-in-water-type skin external agent produced as described above, the formation of oil droplets was visually confirmed and evaluated based on the following evaluation criteria. The results are shown in Tables 1 to 3.

### <Evaluation criteria>

A: oil droplets having appropriate size were formed.
B: fine oil droplets were formed.
C: no oil droplets were formed.

**[Table 1]**

| (% by mass) | | | | | | |
|---|---|---|---|---|---|---|
| Material | Component | | Example 1 | Comparative Example 1 | Comparative Example 2 | Comparative Example 3 |
| A | | Sodium gluconate | 0.3 | 0.3 | 0.3 | 0.3 |
| B | (c2) | 2-Methyl-1,3-propanediol | 3.0 | 3.0 | 3.0 | 3.0 |
| | (b) | Gellan gum | 0.05 | 0.05 | 0.05 | 0.05 |
| C | (a) | Na tocopherol phosphate | 2.0 | 2.0 | 2.0 | 2.0 |
| | (c1) | 1,2-Hexanediol | 4.0 | 4.0 | 4.0 | 4.0 |
| D | | NaCl | 0.4 | 0.4 | 0.4 | 0.4 |
| E | (d) | Propanediol dicaprylate/dicaprate | 0.2 | | | |
| | | Polyglyceryl-2 isostearate | | 0.2 | | |
| | | Polyglyceryl-2 diisostearate | | | 0.2 | |
| | | Polyglyceryl-2 triisostearate | | | | 0.2 |
| | (d) | Triethylhexanoin | | | | |
| | (d) | Caprylic/capric triglyceride | | | | |
| | (d) | Propylene glycol monocaprylate | | | | |
| | (d) | Propylene glycol dicaprylate | | | | |
| | (d) | Meadowfoam oil | | | | |
| | (d) | Jojoba seed oil | | | | |
| | (d) | Macadamia nut oil | | | | |
| Purified water | | | Remainder | Remainder | Remainder | Remainder |
| Total | | | 100 | 100 | 100 | 100 |
| Formation of oil droplets | | | B | C | C | C |

**[Table 2]**

| (% by mass) | | | | | | | |
|---|---|---|---|---|---|---|---|
| Materia l | Component | | Example 2 | Example 3 | Example 4 | Example 5 | Example 6 |
| A | | Sodium gluconate | 0.3 | 0.3 | 0.3 | 0.3 | 0.3 |
| B | (c2) | 2-Methyl-1,3-propanediol | 3.0 | 3.0 | 3.0 | 3.0 | 3.0 |
| | (b) | Gellan gum | 0.05 | 0.05 | 0.05 | 0.05 | 0.05 |
| C | (a) | Na tocopherol phosphate | 2.0 | 2.0 | 2.0 | 2.0 | 2.0 |
| | (c1) | 1,2-Hexanediol | 4.0 | 4.0 | 4.0 | 4.0 | 4.0 |
| D | | NaCl | 0.4 | 0.4 | 0.4 | 0.4 | 0.4 |
| E | (d) | Propanediol dicaprylate/dicaprat e | | | | | |
| | | Polyglyceryl-2 isostearate | | | | | |
| | | Polyglyceryl-2 diisostearate | | | | | |
| | | Polyglyceryl-2 triisostearate | | | | | |
| | (d) | Triethylhexanoin | 0.2 | | | | |
| | (d) | Caprylic/capric triglyceride | | 0.2 | | | |
| | (d) | Propylene glycol monocaprylate | | | 0.2 | | |
| | (d) | Propylene glycol dicaprylate | | | | 0.2 | |
| | (d) | Meadowfoam oil | | | | | 0.2 |
| | (d) | Jojoba seed oil | | | | | |
| | (d) | Macadamia nut oil | | | | | |
| Purified water | | | Remainder | Remainder | Remainder | Remainder | Remainder |
| Total | | | 100 | 100 | 100 | 100 | 100 |
| Formation of oil droplets | | | A | A | B | A | A |

**[Table 3]**

| (% by mass) | | | | |
|---|---|---|---|---|
| Material | Component | | Example 7 | Example 8 |
| A | | Sodium gluconate | 0.3 | 0.3 |
| B | (c2) | 2-Methyl-1,3-propanediol | 3.0 | 3.0 |
| | (b) | Gellan gum | 0.05 | 0.05 |
| C | (a) | Na tocopherol phosphate | 2.0 | 2.0 |
| | (c1) | 1,2-Hexanediol | 4.0 | 4.0 |
| D | | NaCl | 0.4 | 0.4 |
| E | (d) | Propanediol dicaprylate/dicaprate | | |
| | | Polyglyceryl-2 isostearate | | |
| | | Polyglyceryl-2 diisostearate | | |
| | | Polyglyceryl-2 triisostearate | | |
| | (d) | Triethylhexanoin | | |
| | (d) | Caprylic/capric triglyceride | | |
| | (d) | Propylene glycol monocaprylate | | |
| | (d) | Propylene glycol dicaprylate | | |
| | (d) | Meadowfoam oil | | |
| | (d) | Jojoba seed oil | 0.2 | |
| | (d) | Macadamia nut oil | | 0.2 |
| Purified water | | | Remainder | Remainder |
| Total | | | 100 | 100 |
| Formation of oil droplets | | | A | A |

In a case where polyglyceryl-2 isostearate, polyglyceryl-2 diisostearate, or polyglyceryl-2 triisostearate, which did not correspond to the component (d), was used as the material E (oil component) (Comparative Examples 1 to 3), the oil-in-water-type skin external agent was cloudy and no oil droplets were formed. On the other hand, in a case where triethylhexanoin, caprylic/capric triglyceride, propylene glycol dicaprylate, meadowfoam oil, jojoba seed oil, or macadamia nut oil, which was the component (d), was used as the material E (Examples 2, 3, and 5 to 8), oil droplets having an appropriate size were formed.

### (Examples 9 to 13) (reference examples)

### [Production of oil-in-water-type skin external agent]

Oil-in-water-type skin external agents of Examples 9 to 13 were produced in the same manner as in the production of the oil-in-water-type skin external agent of Example 1 described above, except that compositions shown in Table 4 were used. The units of the numerical values shown in Table 4 are % by mass based on the mass of each skin external agent.

### [Evaluation of oil droplets]

With regard to the oil-in-water-type skin external agent produced as described above, the formation of oil droplets was evaluated in the same manner as described above. The results are shown in Table 4.

### [Evaluation of transparency of aqueous phase]

With regard to the oil-in-water-type skin external agent produced as described above, the transparency of the aqueous phase was visually confirmed and evaluated based on the following evaluation criteria. The results are shown in Table 4.

### <Evaluation criteria>

A: transparent
B: translucent
C: opaque

**[Table 4]**

| (% by mass) | | | | | | | |
|---|---|---|---|---|---|---|---|
| Material | Component | | Example 9 | Example 10 | Example 11 | Example 12 | Example 13 |
| A | | Sodium gluconate | 0.3 | 0.3 | 0.3 | 0.3 | 0.3 |
| B | (c2) | 2-Methyl-1,3-propanediol | 3.0 | 3.0 | 3.0 | 3.0 | 3.0 |
| | (b) | Gellan gum | 0.05 | 0.05 | 0.05 | 0.05 | 0.05 |
| C | (a) | Na tocopherol phosphate | 2.0 | 2.0 | 2.0 | 2.0 | 2.0 |
| | (c1) | 1,2-Hexanediol | 4.0 | | 4.0 | | 1.5 |
| | (cl) | Pentylene glycol | | 3.0 | | 3.0 | 1.5 |
| D | | NaCl | 0.4 | 0.4 | 0.4 | 0.4 | 0.4 |
| E | (d) | Caprylic/capric triglyceride | 2.0 | 2.0 | | | |
| | (d) | Macadamia nut oil | | | 2.0 | 2.0 | 2.0 |
| Preservative (phenoxyethanol) | | | 0.4 | 0.4 | 0.4 | 0.4 | 0.4 |
| Purified water | | | Remainder | Remainder | Remainder | Remainder | Remainder |
| Total | | | 100 | 100 | 100 | 100 | 100 |
| Formation of oil droplets | | | A | A | A | A | A |
| Transparency of aqueous phase | | | A | B | A | B | A |

As the dihydric or trihydric alcohol of the material C, in any case where 1,2-hexanediol was used (Examples 9 and 11), where pentylene glycol was used (Examples 10 and 12), where 1,2-hexanediol and pentylene glycol were used in combination (Example 13), oil droplets having an appropriate size were formed. However, in a case where only pentylene glycol was used as the dihydric or trihydric alcohol of the material C, the aqueous phase was slightly translucent.

### (Examples 14 to 19 (reference examples) and Comparative Examples 4 and 5)

### [Production of oil-in-water-type skin external agent]

Oil-in-water-type skin external agents of Examples 14 to 19 and Comparative Examples 4 and 5 were produced in the same manner as in the production of the oil-in-water-type skin external agent of Example 1 described above, except that compositions shown in Tables 5 and 6 were used. The units of the numerical values shown in Tables 5 and 6 are % by mass based on the mass of each skin external agent.

### [Evaluation of oil droplets and evaluation of transparency of aqueous phase]

With regard to the oil-in-water-type skin external agent produced as described above, the formation of oil droplets and the transparency of the aqueous phase were evaluated in the same manner as described above. The results are shown in Tables 5 and 6.

**[Table 5]**

| (% by mass) | | | | | | |
|---|---|---|---|---|---|---|
| Material | Component | | Example 14 | Example 15 | Example 16 | Comparative Example 4 |
| A | | Sodium gluconate | 0.3 | 0.3 | 0.3 | 0.3 |
| B | (c2) | 2-Methyl-1,3-propanediol | 3.0 | 3.0 | 3.0 | 3.0 |
| | | ADEKA NOL GT-930 | | | | 0.05 |
| | (b) | Gellan gum | 0.05 | 0.05 | 0.05 | |
| C | (a) | Na tocopherol phosphate | 0.5 | 1.0 | 2.0 | 2.0 |
| | (c1) | 1,2-Hexanediol | 4.0 | 4.0 | 4.0 | 4.0 |
| D | | NaCl | 0.4 | 0.4 | 0.4 | 0.4 |
| E | (d) | Propanediol dicaprylate/dicaprate | 1.0 | 1.0 | 1.0 | 1.0 |
| | (d) | Caprylic/capric triglyceride | 1.0 | 1.0 | 1.0 | 1.0 |
| | (d) | Macadamia nut oil | | | | |
| Preservative (phenoxyethanol) | | | 0.4 | 0.4 | 0.4 | 0.4 |
| pH adjuster (citric acid) | | | Moderate amount | Moderate amount | Moderate amount | |
| Purified water | | | Remainder | Remainder | Remainder | Remainder |
| Total | | | 100 | 100 | 100 | 100 |
| pH | | | 6.5 | 6.4 | 6.4 | Unadjusted |
| Formation of oil droplets | | | A | A | A | C |
| Transparency of aqueous phase | | | A | A | A | - |

**[Table 6]**

| (% by mass) | | | | | | |
|---|---|---|---|---|---|---|
| Material | Component | | Example 17 | Example 18 | Example 19 | Comparative Example 5 |
| A | | Sodium gluconate | 0.3 | 0.3 | 0.3 | 0.3 |
| B | (c2) | 2-Methyl-1,3-propanediol | 3.0 | 3.0 | 3.0 | 3.0 |
| | | ADEKA NOL GT-930 | | | | 0.05 |
| | (b) | Gellan gum | 0.05 | 0.05 | 0.05 | |
| C | (a) | Na tocopherol phosphate | 0.5 | 1.0 | 2.0 | 2.0 |
| | (c1) | 1,2-Hexanediol | 4.0 | 4.0 | 4.0 | 4.0 |
| D | | NaCl | 0.4 | 0.4 | 0.4 | 0.4 |
| E | (d) | Dicapric acid propanediol | | | | |
| | (d) | Caprylic/capric triglyceride | | | | |
| | (d) | Macadamia nut oil | 2.0 | 2.0 | 2.0 | 2.0 |
| Preservative (phenoxyethanol) | | | 0.4 | 0.4 | 0.4 | 0.4 |
| pH adjuster (citric acid) | | | Moderate amount | Moderate amount | Moderate amount | |
| Purified water | | | Remainder | Remainder | Remainder | Remainder |
| Total | | | 100 | 100 | 100 | 100 |
| pH | | | 6.3 | 6.3 | 6.4 | Unadjusted |
| Formation of oil droplets | | | A | A | A | C |
| Transparency of aqueous phase | | | A | A | A | - |

In Comparative Examples 4 and 5 in which ADEKA NOL (registered trademark) GT-930 (manufactured by ADEKA CORPORATION), which was a hydrophobically modified polyether urethane polymer, was used as the thickener of the material B, the oil-in-water-type skin external agent did not thicken at all and no oil droplets were formed. On the other hand, in Examples 14 to 19 in which gellan gum was used as the thickener of the material B, at any blending amount of Na tocopherol phosphate, oil droplets were formed and the aqueous phase was transparent.

### (Examples 20 to 23 (Examples 20, 22 and 23 are reference examples) and Comparative Examples 6 to 8)

### [Production of oil-in-water-type skin external agent]

Oil-in-water-type skin external agents of Examples 20 to 23 and Comparative Examples 6 to 8 were produced in the same manner as in the production of the oil-in-water-type skin external agent of Example 1 described above, except that compositions shown in Tables 7 and 8 were used. The units of the numerical values shown in Tables 7 and 8 are % by mass based on the mass of each skin external agent.

### [Evaluation of oil droplets and evaluation of transparency of aqueous phase]

With regard to the oil-in-water-type skin external agent produced as described above, the formation of oil droplets and the transparency of the aqueous phase were evaluated in the same manner as described above. The results are shown in Tables 7 and 8.

### [Evaluation of viscosity (thickening)]

With regard to the oil-in-water-type skin external agent produced as described above, the viscosity (thickening) was visually confirmed and evaluated based on the following evaluation criteria. The results are shown in Tables 7 and 8.

### <Evaluation criteria>

A: there was moderate thickening.
B: there was a slight thickening.
C: oil-in-water-type skin external agent did not thicken.

### [Evaluation of stability]

The oil-in-water-type skin external agents of Examples 20 to 23 were allowed to stand at 4°C, 25°C, 40°C, or 50°C for 4 weeks. Thereafter, the appearance of each oil-in-water-type skin external agent was visually observed and evaluated based on the following evaluation criteria. The results are shown in Table 8. In the following evaluation criteria, "creaming" means a phenomenon in which the oil phase floats in the upper layer.

### <Evaluation criteria>

A: creaming did not occur.
B: creaming occurred.

**[Table 7]**

| (% by mass) | | | | | |
|---|---|---|---|---|---|
| Material | Component | | Comparative Example 6 | Comparative Example 7 | Comparative Example 8 |
| A | | Sodium gluconate | 0.3 | 0.3 | 0.3 |
| B | (c2) | 2-Methyl-1,3-propanediol | 3.0 | 3.0 | 3.0 |
| | | ADEKA NOL GT-930 | | 0.1 | |
| | (b) | Gellan gum | | | |
| | (f) | Tamarind gum | | | 0.05 |
| | | Glucomannan | 0.05 | | |
| | | Xanthan gum | | | |
| | | Locust bean gum | | | |
| C | (a) | Na tocopherol phosphate | 2.0 | 2.0 | 2.0 |
| | (c1) | 1,2-Hexanediol | 4.0 | 4.0 | 4.0 |
| D | | NaCl | 0.4 | 0.4 | 0.4 |
| E | (d) | Propanediol dicaprylate/dicaprate | 1.0 | 1.0 | 1.0 |
| | (d) | Caprylic/capric triglyceride | 1.0 | 1.0 | 1.0 |
| Coloring agent (Sudan III) | | | Moderate amount | Moderate amount | Moderate amount |
| Preservative (phenoxyethanol) | | | 0.4 | 0.4 | 0.4 |
| pH adjuster (citric acid) | | | | | |
| Purified water | | | Remainder | Remainder | Remainder |
| Total | | | 100 | 100 | 100 |
| pH | | | Unadjusted | Unadjusted | Unadjusted |
| Formation of oil droplets | | | B | C | C |
| Transparency of aqueous phase | | | C | A | A |
| Viscosity (thickening) | | | B | C | C |

**[Table 8]**

| (% by mass) | | | | | | | |
|---|---|---|---|---|---|---|---|
| Material | Component | | | Example 20 | Example 21 | Example 22 | Example 23 |
| A | | Sodium gluconate | | 0.3 | 0.3 | 0.3 | 0.3 |
| B | (c2) | 2-Methyl-1,3-propanediol | | 3.0 | 3.0 | 3.0 | 3.0 |
| | | ADEKA NOL GT-930 | | 0.05 | | | |
| | (b) | Gellan gum | | 0.05 | 0.05 | 0.05 | 0.05 |
| | (f) | Tamarind gum | | | 0.05 | | |
| | | Glucomannan | | | | | |
| | | Xanthan gum | | | | 0.05 | |
| | | Locust bean gum | | | | | 0.05 |
| C | (a) | Na tocopherol phosphate | | 2.0 | 2.0 | 2.0 | 2.0 |
| | (c1) | 1,2-Hexanediol | | 4.0 | 4.0 | 4.0 | 4.0 |
| D | | NaCl | | 0.4 | 0.4 | 0.4 | 0.4 |
| E | (d) | Propanediol dicaprylate/dicaprate | | 1.0 | 1.0 | 1.0 | 1.0 |
| | (d) | Caprylic/capric triglyceride | | 1.0 | 1.0 | 1.0 | 1.0 |
| Coloring agent (Sudan III) | | | | Moderate amount | Moderate amount | Moderate amount | Moderate amount |
| Preservative (phenoxyethanol) | | | | 0.4 | 0.4 | 0.4 | 0.4 |
| pH adjuster (citric acid) | | | | | | | |
| Purified water | | | | Remainder | Remainder | Remainder | Remainder |
| Total | | | | 100 | 100 | 100 | 100 |
| pH | | | | Unadjusted | Unadjusted | Unadjusted | Unadjusted |
| Formation of oil droplets | | | | A | A | A | A |
| Transparency of aqueous phase | | | | B | B | B | B |
| Viscosity (thickening) | | | | A | A | A | A |
| Stability | | | 4°C | B | A | B | B |
| | | | 25°C | B | A | B | B |
| | | | 40°C | B | A | B | B |
| | | | 50°C | B | A | B | B |

In Examples 20 to 23 in which gellan gum and other thickeners were used in combination as the thickener of the material B, an oil-in-water-type skin external agent having a moderate thickening could be prepared. On the other hand, in a case where gellan gum was not used as the thickener of the material B (Comparative Examples 6 to 8), there was no thickening or the aqueous phase was opaque.

In addition, from the results of the stability test, it was confirmed that, among Examples 20 to 23 the oil-in-water-type skin external agent of Example 21 was the most stable.

### (Examples 24 to 30)

### [Production of oil-in-water-type skin external agent]

Oil-in-water-type skin external agents of Examples 24 to 30 were produced in the same manner as in the production of the oil-in-water-type skin external agent of Example 1 described above, except that compositions shown in Tables 9 and 10 were used. The units of the numerical values shown in Tables 9 and 10 are % by mass based on the mass of each skin external agent.

### [Evaluation of oil droplets, evaluation of transparency of aqueous phase, evaluation of viscosity, and evaluation of stability]

With regard to the oil-in-water-type skin external agent produced as described above, the formation of oil droplets, the transparency of the aqueous phase, the viscosity, and the stability were evaluated in the same manner as described above. The results are shown in Tables 9 and 10.

### [Measurement of particle size of oil droplets]

Among the oil-in-water-type skin external agent produced as described above, the particle size of oil droplets was measured for Examples 24 to 26 and Example 30. Approximately 50 µL of the agent was dropped onto a 1-hole hole slide glass, and the 1-hole hole slide glass was covered with a cover glass to prepare a measurement sample. A digital microscope (VHX-5000, KEYENCE CORPORATION) was used for the measurement, and the average particle size of oil droplets was determined by analyzing an image taken at a magnification of 100 under the transmission condition by the measurement function attached to the VHX-5000. The average particle size of oil droplets was the average value of 200 to 600 oil droplets. The results are shown in Tables 9 and 10.

**[Table 9]**

| (% by mass) | | | | | | |
|---|---|---|---|---|---|---|
| Material | Component | | | Example 24 | Example 25 | Example 26 |
| A | | Sodium gluconate | | 0.3 | 0.3 | 0.3 |
| B | (c2) | 2-Methyl-1,3-propanediol | | 1.5 | 2.5 | 2 |
| | (c2) | Glycerin | | 1.0 | 1.0 | 1.5 |
| | (b) | Gellan gum | | 0.05 | 0.05 | 0.05 |
| | (f) | Tamarind gum | | 0.05 | 0.05 | 0.05 |
| C | (a) | Na tocopherol phosphate | | 2.0 | 2.0 | 2.0 |
| | (c1) | 1,2-Hexanediol | | 4.0 | 4.0 | 4.0 |
| D | | NaCl | | 0.4 | 0.4 | 0.4 |
| E | (d) | Propanediol dicaprylate/dicaprate | | 1.0 | 1.0 | 1.0 |
| | (d) | Caprylic/capric triglyceride | | 1.0 | 1.0 | 1.0 |
| Coloring agent (Sudan III) | | | | Moderate amount | Moderate amount | Moderate amount |
| Preservative (phenoxyethanol) | | | | 0.4 | 0.4 | 0.4 |
| pH adjuster (citric acid) | | | | | | |
| Purified water | | | | Remainder | Remainder | Remainder |
| Total | | | | 100 | 100 | 100 |
| pH | | | | Unadjusted | Unadjusted | Unadjusted |
| Formation of oil droplets | | | | B | B | A |
| Transparency of aqueous phase | | | | A | A | A |
| Viscosity (thickening) | | | | C | B | B |
| Stability | | | 4°C | A | A | A |
| | | | 25°C | A | A | A |
| | | | 40°C | A | A | A |
| | | | 50°C | A | A | A |
| Average particle size of oil droplets (µm) | | | | 136 | 135 | 124 |

**[Table 10]**

| (% by mass) | | | | | | | |
|---|---|---|---|---|---|---|---|
| Material | Component | | | Example 27 | Example 28 | Example 29 | Example 30 |
| A | | Sodium gluconate | | 0.3 | 0.3 | 0.3 | 0.3 |
| B | (c2) | 2-Methyl-1,3-propanediol | | 3.0 | 3.0 | 3.0 | 2.5 |
| | (c2) | Glycerin | | 0.5 | 0.25 | 0.1 | 1.0 |
| | (b) | Gellan gum | | 0.05 | 0.05 | 0.05 | 0.05 |
| | (f) | Tamarind gum | | 0.05 | 0.05 | 0.05 | 0.05 |
| C | (a) | Na tocopherol phosphate | | 2.0 | 2.0 | 2.0 | 2.0 |
| | (c1) | 1,2-Hexanediol | | 4.0 | 4.0 | 4.0 | 4.0 |
| D | | NaCl | | 0.4 | 0.4 | 0.4 | 0.4 |
| E | (d) | Propanediol dicaprylate/dicaprate | | 1.0 | 1.0 | 1.0 | 0.5 |
| | (d) | Caprylic/capric triglyceride | | 1.0 | 1.0 | 1.0 | 0.5 |
| Coloring agent (Sudan III) | | | | Moderate amount | Moderate amount | Moderate amount | Moderate amount |
| Preservative (phenoxyethanol) | | | | 0.4 | 0.4 | 0.4 | 0.4 |
| pH adjuster (citric acid) | | | | | | | |
| Purified water | | | | Remainder | Remainder | Remainder | Remainder |
| Total | | | | 100 | 100 | 100 | 100 |
| pH | | | | Unadjusted | Unadjusted | Unadjusted | Unadjusted |
| Formation of oil droplets | | | | A | A | A | A |
| Transparency of aqueous phase | | | | A | A | A | A |
| Viscosity (thickening) | | | | A | A | A | A |
| Stability | | | 4°C | B | B | B | A |
| | | | 25°C | B | B | B | A |
| | | | 40°C | B | B | B | A |
| | | | 50°C | B | B | B | A |
| Average particle size of oil droplets (µm) | | | | - | - | - | 127 |

In any one of Examples 24 to 30, it was possible to form an oil-in-water-type skin external agent which has a moderate thickening and in which the aqueous phase is transparent. Among these, it was confirmed that the oil-in-water-type skin external agent of Example 30 had an aesthetic appearance from the oil droplets having an appropriate size and the transparency of the aqueous phase, had a moderate thickening, and was the most stable.

Table 11 shows the product names and the like of each component used in Examples and Comparative Examples described above.

**[Table 11]**

| Component name | Product name | Manufacturer name |
|---|---|---|
| Sodium gluconate | | Wako Pure Chemical Corporation |
| Na tocopherol phosphate | TPNa | SHOWA DENKO K.K. |
| 1,2-Hexanediol | KMO-6 | Kankohsha co., ltd. |
| Pentylene glycol | HYDROLITE-5 | Iwase Cosfa Co., Ltd. |
| Glycerin | GLYCERIN (COSMETIC GRADE) | Kao Corporation |
| 2-Methyl-1,3-propanediol | | Tokyo Chemical Industry Co., Ltd. |
| Gellan gum | KELCOGEL Gellan Gum CG-LA | CP Kelco |
| Tamarind gum | GLYLOID 6C | DSP GOKYO FOOD & CHEMICAL Co., Ltd. |
| Glucomannan | RHEOLEX (registered trademark) RS | Shimizu Chemical Corporation |
| Xanthan gum | KELTROL (registered trademark) CG-BT | CP Kelco |
| Locust bean gum | GENUGUM RL- 200Z | SANSHO Co., Ltd. |
| Propanediol dicaprylate/dicaprate | SALACOS PR-85 | The Nisshin OilliO Group, Ltd. |
| Polyglyceryl-2 isostearate | Cosmol (registered trademark) 41V | The Nisshin OilliO Group, Ltd. |
| Polyglyceryl-2 diisostearate | Cosmol (registered trademark) 42V | The Nisshin OilliO Group, Ltd. |
| Polyglyceryl-2 triisostearate | Cosmol (registered trademark) 43V | The Nisshin OilliO Group, Ltd. |
| Triethylhexanoin | T.I.O | The Nisshin OilliO Group, Ltd. |
| Caprylic/capric triglyceride | Triester F-810 | Nikko Chemicals Co., Ltd. |
| Propylene glycol monocaprylate | NIKKOL Sefsol218 | Nikko Chemicals Co., Ltd. |
| Propylene glycol dicaprylate | NIKKOL Sefsol228 | Nikko Chemicals Co., Ltd. |
| Meadowfoam oil | Meadowfoam oil | Nikko Chemicals Co., Ltd. |
| Jojoba seed oil | Jojoba oil | Nikko Chemicals Co., Ltd. |
| Macadamia nut oil | Macadamia nut oil | Nikko Chemicals Co., Ltd. |
| Sudan III | | Junsei Chemical Co., Ltd. |
| Phenoxyethanol | Phenoxyethanol | SEIWA KASEI Co., Ltd. |

### [Industrial Applicability]

According to the present invention, an oil-in-water-type skin external agent containing sodium tocopherol phosphate as an active ingredient, in which oil droplets that are visible are dispersed and excellent aesthetic appearance is exhibited, is provided.

## Claims

1. An oil-in-water-type skin external agent comprising:
(a) 0.1 to 3.0 % by mass of sodium tocopherol phosphate;
(b) 0.01% to 1.0% by mass of gellan gum;
(c1) 0.2 to 6.0% by mass of 1,2-hexanediol;
(c2) 0.9% to 4.0% by mass of 2-methyl-1,3-propanediol and 0.13% to 1.6% by mass of glycerin;
(d) 0.1% to 2.0% by mass of propanediol dicaprylate/dicaprate and 0.1% to 2.0% by mass of caprylic/capric triglyceride;
(e) water; and
(f) 0.01% to 1.0% by mass of tamarind gum.

2. The oil-in-water-type skin external agent according to Claim 1,
wherein an oil droplet has an average particle size of 5 to 450 µm.

3. The oil-in-water-type skin external agent according to Claim 1 or 2,
wherein a proportion (mass ratio) of the component (b) and the component (f) in the oil-in-water-type skin external agent is component (b):component (f) = 1:0.5 to 1:2.

## Patentansprüche

1. Mittel zur äußeren Hautanwendung vom Öl-in-Wasser-Typ, umfassend:
(a) 0,1 bis 3,0 Massen-% Natriumtocopherolphosphat,
(b) 0,01 Massen-% bis 1,0 Massen-% Gellan-Gummi,
(c1) 0,2 bis 6,0 Massen-% 1,2-Hexandiol,
(c2) 0,9 Massen-% bis 4,0 Massen-% 2-Methyl-1,3-propandiol und 0,13 Massen-% bis 1,6 Massen-% Glycerin,
(d) 0,1 Massen-% bis 2,0 Massen-% Propandioldicaprylat/dicaprat und 0,1% bis 2,0 Massen-% Capryl-/Caprin-Triglycerid,
(e) Wasser; und
(f) 0,01 Massen-% bis 1,0 Massen-% Tamarinden-Gummi.

2. Mittel zur äußeren Hautanwendung vom Öl-in-Wasser-Typ nach Anspruch 1,
wobei ein Öltröpfchen eine mittlere Teilchengröße von 5 bis 450 µm aufweist.

3. Mittel zur äußeren Hautanwendung vom Öl-in-Wasser-Typ nach Anspruch 1 oder 2,
wobei ein Anteil (Massenverhältnis) der Komponente (b) und der Komponente (f) in dem Mittel zur äußeren Hautanwendung vom Öl-in-Wasser-Typ wie folgt ist: Komponente (b) : Komponente (f) = 1 : 0,5 bis 1 : 2.

## Revendications

1. Agent externe pour la peau de type huile-dans-eau comprenant :
(a) de 0,1 à 3,0 % en masse de phosphate de tocophérol de sodium ;
(b) de 0,01 % à 1,0 % en masse de gomme gellane ;
(c1) de 0,2 % à 6,0 % en masse de 1,2-hexanediol ;
(c2) de 0,9 % à 4,0 % en masse de 2-méthyl-1,3-propanediol et de 0,13 % à 1,6 % en masse de glycérine ;
(d) de 0,1 % à 2,0 % en masse de dicaprylate/dicaprate de propanediol et de 0,1 % à 2,0 % en masse de triglycéride caprylique/caprique ;
(e) de l'eau ; et
(f) de 0,01 % à 1,0 % en masse de gomme de tamarin.

2. Agent externe pour la peau de type huile-dans-eau selon la revendication 1,
dans lequel une gouttelette d'huile présente une taille moyenne de particule de 5 à 450 µm.

3. Agent externe pour la peau de type huile-dans-eau selon la revendication 1 ou 2,
dans lequel la proportion (rapport en masse) du composant (b) et du composant (f) est : composant (b) : composant (f) = 1 : 0,5 à 1 : 2.
